# EUROPEAN PATENT APPLICATION

(11) **EP 1 894 926 A1**
(43) Date of publication of application: **05.03.2008**
(21) Application number: 06013132.3
(22) Date of filing: 26.06.2006
(51) Int. Cl.: C07D 403/12, A61K 31/506

(54) **Polymorphs of moxonidine and processes for preparation therefor**

(71) Applicant: CHEMAGIS LTD., 51200 Bnei-Brak (IL)
(72) Inventor: Adin, Itai, 84684 Beer-Sheva (IL); Iustain, Carmen, 84750 Beer-Sheva (IL); Naddaka, Vladimir, 71338 Lod (IL); Klopfer, Eyal, 64955 Tel Aviv (IL); Arad, Oded, 76303 Rechovot (IL); Kaspi, Joseph, 53201 Givatayim (IL)
(74) Representative: Becker Kurig Straus

(57) **Abstract**

Three crystalline forms of Moxonidine designated as Moxonidine form I, form II and form III and processes for their preparation are disclosed.

## Description

### FIELD OF THE INVENTION

The present invention relates to the solid state chemistry of the drug Moxonidine.

### BACKGROUND OF THE INVENTION

Moxonidine (4-chloro-5-(imidazoline-2-ylamino)-6-methoxy-2-methylpyrimidine), has the structural formula (I) below and is used as an antihypertensive drug.

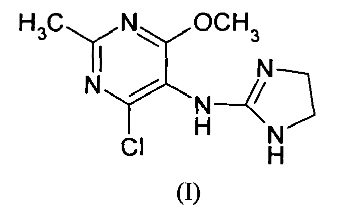

Moxonidine was approved for use in Germany in 1991 and is currently commercially available in Europe, e.g., in Germany, Austria and in the UK.

U.S. patent No. 4,323,570 (hereinafter the '570 patent) describes a method of preparing Moxonidine, wherein in example 3 it is stated that Moxonidine is obtained by crystallization from nitromethane having a melting point of 217-219°C. The hydrochloride salt of Moxonidine is described in example 25, having a melting point of 189°C. However, the solid state chemistry of Moxonidine was not referred to in the '570 patent or in any other prior art literature.

The teaching of European patent applications titled "Improved process for producing Moxonidine" and "Novel purification process of Moxonidine" by the present applicant, filed concurrently on even date are incorporated herein by reference.

The object of the present invention is to provide stable crystalline polymorphs of Moxonidine, which are suitable for use in pharmaceutical formulations and improved processes for preparing these stable, crystalline polymorphs by crystallization from safe solvents that are conveniently used in the pharmaceutical industry.

### BRIEF SUMMARY OF THE INVENTION

The present invention provides crystalline forms of Moxonidine, which are named form I, form II and form III.

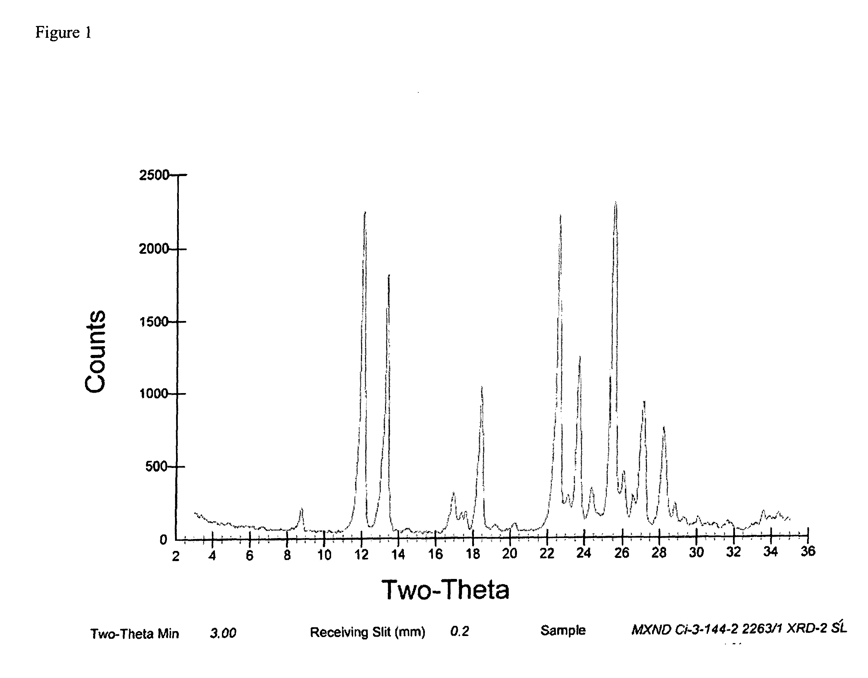

According to one embodiment of the present invention, the crystalline Moxonidine form I produces a unique X-ray powder diffraction pattern (figure 1). The strong diffraction peaks at 12.1, 13.4, 18.4, 22.6, 23.7, 25.5, 27.1 and 28.2±0.2 degrees 2θ are most characteristic of this form.

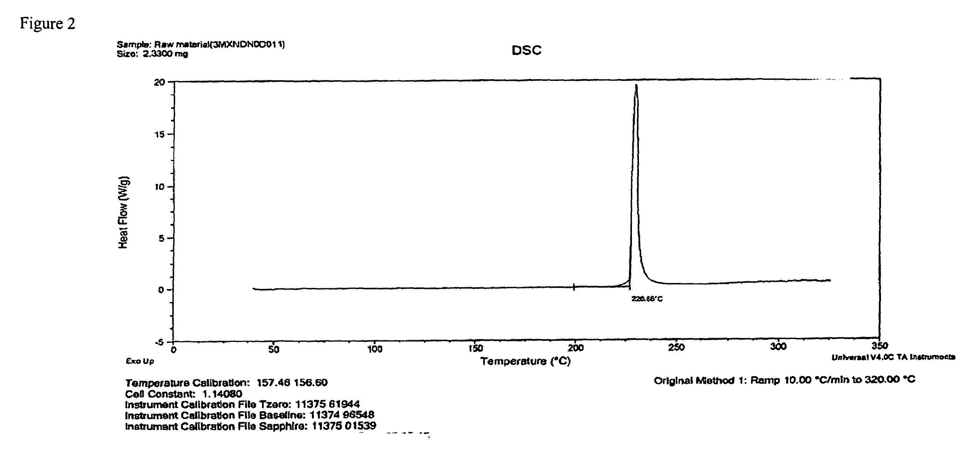

In another embodiment of the present invention, a characteristic DSC curve of the crystalline Moxonidine form I, which contains an exothermic peak at about 227°C, is depicted in figure 2.

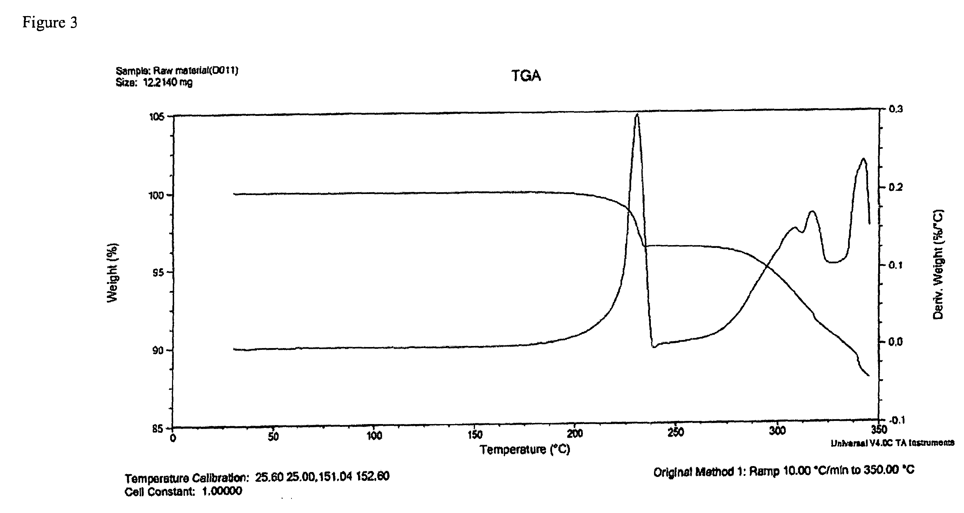

In yet another embodiment of the present invention, characteristic TGA curve of the crystalline Moxonidine form I is depicted in figure 3.

The present invention provides a process for preparing the crystalline Moxonidine form I, which comprises:
slurrying or dissolving Moxonidine, which is obtained by any method known in the art, e.g., by example 1, in the solvent optionally at elevated temperature;
allowing the mixture to cool sufficiently; and
collecting the crystals by filtration and drying.

In a preferred embodiment, the crystalline Moxonidine form I is prepared by slurrying in or crystallizing from a solvent selected from the group consisting of water, glycols wherein a preferred glycol is propylene glycol; polar solvents, wherein preferred polar solvents are dimethyl sulfoxide (DMSO), N,N-dimethylformamide (DMF), N,N-dimethylacetamide (DMA), and N-methyl-2-pyrrolidone (NMP); alcohols, wherein preferred alcohols are methanol, ethanol, 1-propanol, 2-propanol, 1-butanol, 2-butanol, 2-methyl-1-propanol, 3-methyl-1-propanol, and n-amyl-alcohol; R₁COOR₂ esters, while R₁=C₁-C₅ alkyl and R₂=C₁-C₅ alkyl, wherein preferred esters are ethyl formate, methyl acetate, ethyl acetate, n-propyl acetate, isopropyl acetate, n-butyl acetate, isobutyl acetate, and tert-butyl acetate; C₁-C₆ ketones, wherein preferred ketones are acetone, methyl ethyl ketone (MEK), diethyl ketone, methyl propyl ketone, and methyl isobutyl ketone (MIBK); halogenated solvents, wherein a preferred halogenated solvent is dichloromethane; ethers, wherein preferred ethers are diethyl ether, tert-butyl methyl ether, and tetrahydrofuran (THF); aromatic solvents, wherein preferred aromatic solvents are anisole, cumene and toluene; hydrocarbons, wherein preferred hydrocarbons are pentane, hexane, heptane, cyclohexane and methylcyclohexane, and mixtures thereof.

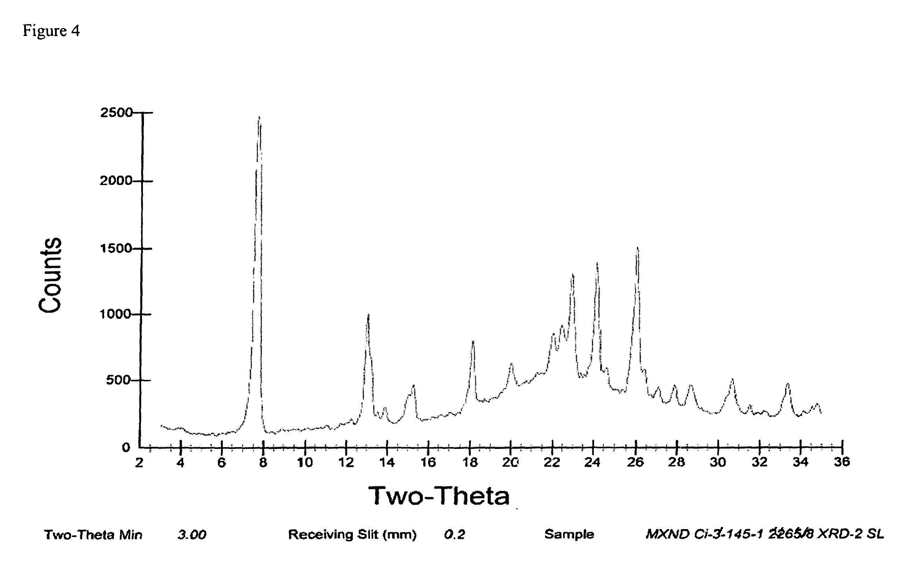

In yet another embodiment of the present invention there is provided a crystalline Moxonidine form II, which produces a unique X-ray powder diffraction pattern (figure 4). The strong diffraction peaks at 7.5, 12.8, 13.0, 18.0, 22.7, 24.0, and 25.8 ±0.2 degrees 2θ are most characteristic of this form.

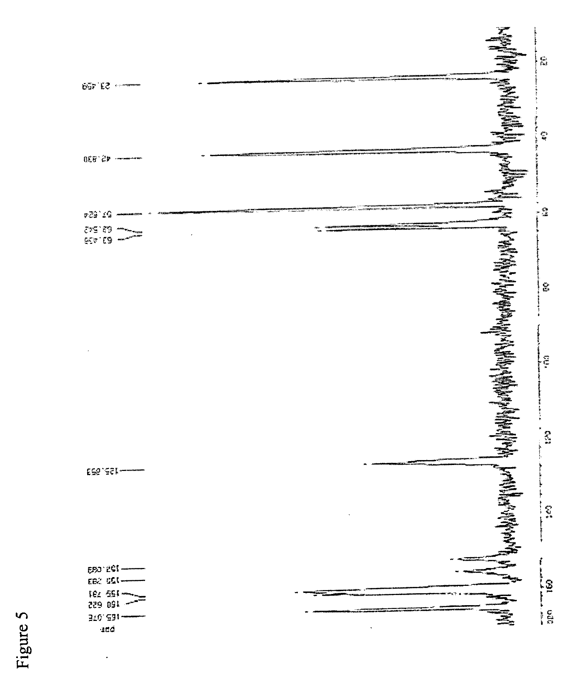

In yet another embodiment of the present invention, the crystalline Moxonidine form II is an ethylene glycol solvate, as determined by the ¹³C NMR spectrum and as depicted in figure 5. The two signals at 62.5 and 63.4 ppm are attributed to ethylene glycol, wherein one of these signals probably arises from a molecule inside the crystal lattice, while the other one might be surface material. It should be noted that the two carbons of ethylene glycol are related by symmetry, therefore only a difference in the chemical environment, caused by inclusion in the crystal lattice, can cause these two carbons to exhibit different chemical shifts.

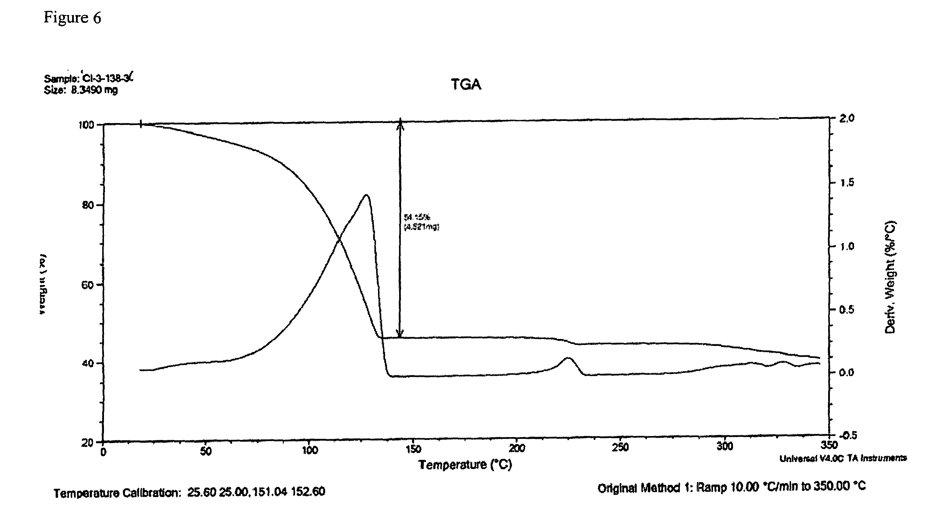

In yet another embodiment of the present invention, characteristic TGA curve of the crystalline Moxonidine form II is depicted in figure 6.

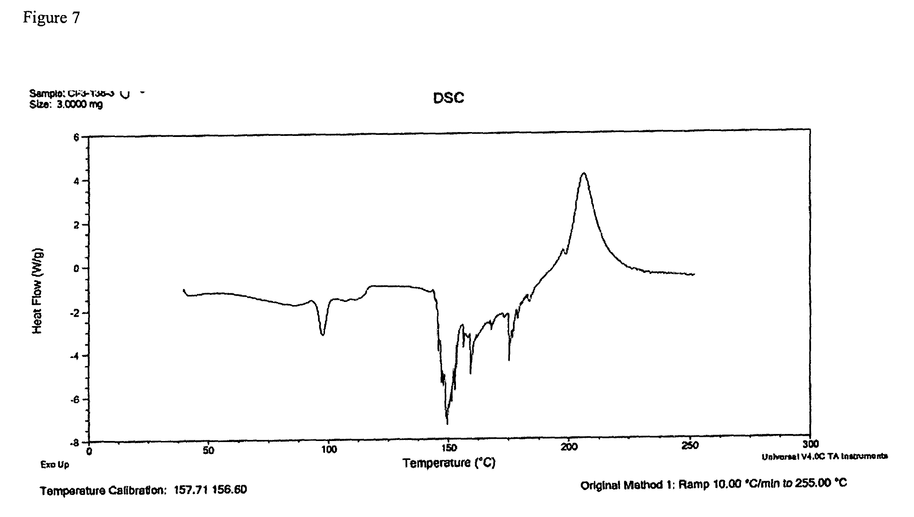

In yet another embodiment of the present invention, characteristic DSC curve of the crystalline Moxonidine form II is depicted in figure 7.

In another preferred embodiment, the present invention provides a process for preparing the crystalline Moxonidine form II comprising crystallizing the crude Moxonidine, which is obtained by any method known in the art, e.g., by example 1, from ethylene glycol and recovering the crystalline form.

The process for preparing crystalline Moxonidine form II, by crystallizing from ethylene glycol, comprising:
mixing Moxonidine, which is obtained by any method known in the art, e.g.,
by example 1, with ethylene glycol and heating to dissolution;
allowing the mixture to cool sufficiently; and
filtering off the thus obtained slurry and obtaining a solid;

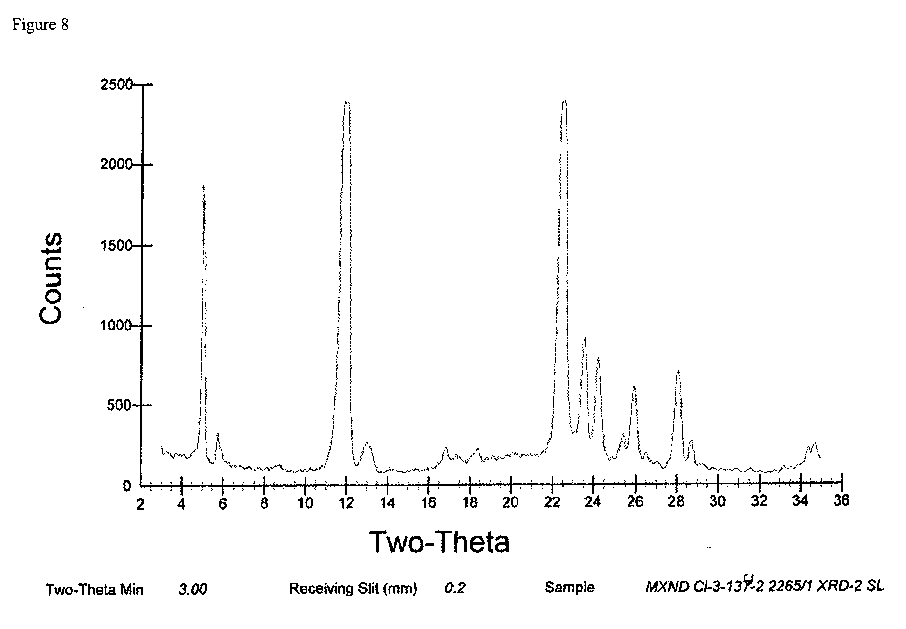

Crystalline Moxonidine form III produces a unique X-ray powder diffraction pattern (figure 8). The strong diffraction peaks at 5.0, 12.0, and 22.5±0.2 degrees 2θ are most characteristic of this form.

In a preferred embodiment of the present invention, the crystalline Moxonidine form III is an n-pentylamine solvate.

The process for preparing crystalline Moxonidine form III, by crystallizing from n-pentylamine, comprising:
mixing Moxonidine, which is obtained by any method known in the art, e.g., by example 1, with n-pentylamine and heating to dissolution;
allowing the mixture to cool sufficiently; and
filtering off the thus obtained slurry and obtaining a solid;

In another embodiment of the present invention, a process is provided for preparing the crystalline Moxonidine form I by heating the crystalline Moxonidine form II or form III at elevated temperature.

### BRIEF DESCRIPTION OF FIGURES

Figure 1 - depicts the X-ray powder diffraction pattern of the crystalline Moxonidine form I
Figure 2 - depicts the DSC curve of the crystalline Moxonidine form I
Figure 3 - depicts the TGA curve of the crystalline Moxonidine form I
Figure 4 - depicts the X-ray powder diffraction pattern of the crystalline Moxonidine form II
Figure 5 - depicts the ¹³C NMR spectrum of the crystalline Moxonidine form II
Figure 6 - depicts the TGA curve of the crystalline Moxonidine form II
Figure 7 - depicts the DSC curve of the crystalline Moxonidine form II
Figure 8 - depicts the X-ray powder diffraction pattern of the crystalline Moxonidine form III

### DESCRIPTION OF THE PREFERRED EMBODIMENTS

The present invention provides crystalline forms of Moxonidine, which are named form I, form II and form III.

According to one embodiment of the present invention, the crystalline Moxonidine form I produces a unique X-ray powder diffraction pattern (figure 1 and table 1). The strong diffraction peaks at 12.1, 13.4, 18.4, 22.6, 23.7, 25.5, 27.1 and 28.2±0.2 degrees 2θ are most characteristic of this form.

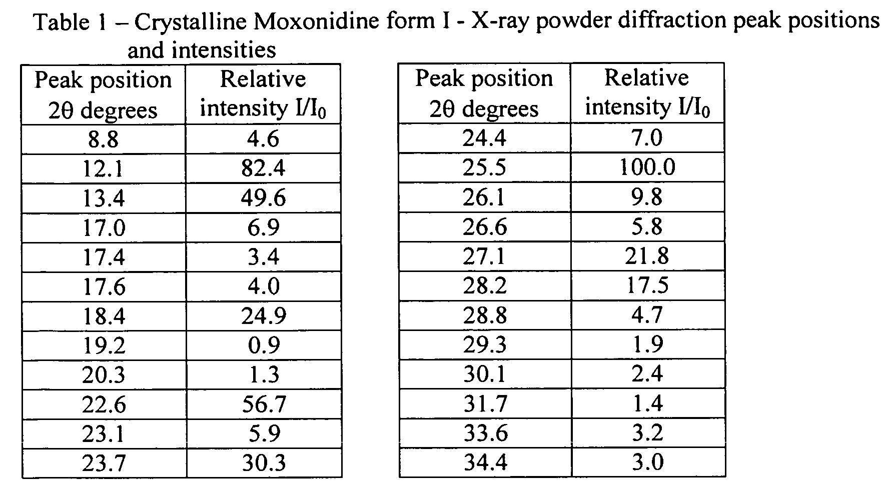

In another embodiment of the present invention, the characteristic DSC curve of the crystalline Moxonidine form I, which contains an exothermic peak at about 227°C, is depicted in figure 2.

In yet another embodiment of the present invention, characteristic TGA curve of the crystalline Moxonidine form I is depicted in figure 3.

The present invention provides a process for preparing Moxonidine crystalline form I, which comprises:
slurrying or dissolving Moxonidine, which is obtained by any method known
in the art, e.g., by example 1, in the solvent optionally at elevated temperature;
allowing the mixture to cool sufficiently; and
collecting the crystals by filtration and drying.

In a preferred embodiment, the crystalline Moxonidine form I is prepared by slurrying in or crystallizing from a solvent selected from the group consisting of water, glycols wherein a preferred glycol is propylene glycol; polar solvents, wherein preferred polar solvents are dimethyl sulfoxide (DMSO), N,N-dimethylformamide (DMF), N,N-dimethylacetamide (DMA), and N-methyl-2-pyrrolidone (NMP); alcohols, wherein preferred alcohols are methanol, ethanol, 1-propanol, 2-propanol, 1-butanol, 2-butanol, 2-methyl-1-propanol, 3-methyl-1-propanol, and n-amyl-alcohol; R₁COOR₂ esters, while R₁=C₁-C₅ alkyl and R₂=C₁-C₅ alkyl, wherein preferred esters are ethyl formate, methyl acetate, ethyl acetate, n-propyl acetate, isopropyl acetate, n-butyl acetate, isobutyl acetate, and tert-butyl acetate; C₁-C₆ ketones, wherein preferred ketones are acetone, methyl ethyl ketone (MEK), diethyl ketone, methyl propyl ketone, and methyl isobutyl ketone (MIBK); halogenated solvents, wherein a preferred halogenated solvent is dichloromethane; ethers, wherein preferred ethers are diethyl ether, tert-butyl methyl ether, and tetrahydrofuran (THF); aromatic solvents, wherein preferred aromatic solvents are anisole, cumene and toluene; hydrocarbons, wherein preferred hydrocarbons are pentane, hexane, heptane, cyclohexane and methylcyclohexane, and mixtures thereof.

In yet another embodiment of the present invention, there is provided a crystalline Moxonidine form II, which produces a unique X-ray powder diffraction pattern (figure 4 and table 2). The strong diffraction peaks at 7.5, 12.8, 13.0, 18.0, 22.7, 24.0, and 25.8 ±0.2 degrees 2θ are most characteristic of this form.

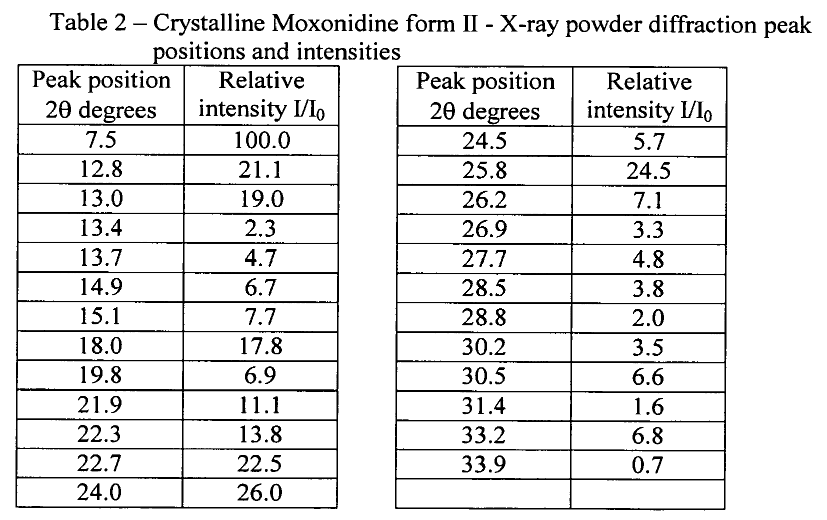

In another embodiment of the present invention, the crystalline Moxonidine form II is an ethylene glycol solvate, as determined by the ¹³C NMR spectrum and as depicted in figure 5. The two signals at 62.5 and 63.4 ppm are attributed to ethylene glycol, wherein one of these signals probably arises from a molecule inside the crystal lattice, while the other one might be surface material. It should be noted that the two carbons of ethylene glycol are related by symmetry, therefore only a difference in the chemical environment, caused by inclusion in the crystal lattice, can cause these two carbons to exhibit different chemical shifts.

In yet another embodiment of the present invention, characteristic TGA curve of the crystalline Moxonidine form II is depicted in figure 6, showing mainly the free ethylene glycol being evaporated.

In yet another embodiment of the present invention, characteristic DSC curve of the crystalline Moxonidine form II is depicted in figure 7.

In another preferred embodiment, the present invention provides a process for preparing the crystalline Moxonidine form II comprising crystallizing the crude Moxonidine, which is obtained by any method known in the art, e.g., by example 1, from ethylene glycol and recovering the crystalline form.

The process for preparing the crystalline Moxonidine form II, by crystallizing from ethylene glycol, comprises:
mixing Moxonidine, which is obtained by any method known in the art, e.g., by example 1, with ethylene glycol and heating to dissolution;
allowing the mixture to cool sufficiently; and
filtering off the thus obtained slurry and obtaining a solid;
   Crystalline Moxonidine form III produces a unique X-ray powder diffraction pattern (figure 8 and table 3). The strong diffraction peaks at 5.0, 12.0, and 22.5±0.2 degrees 2θ are most characteristic of this form.

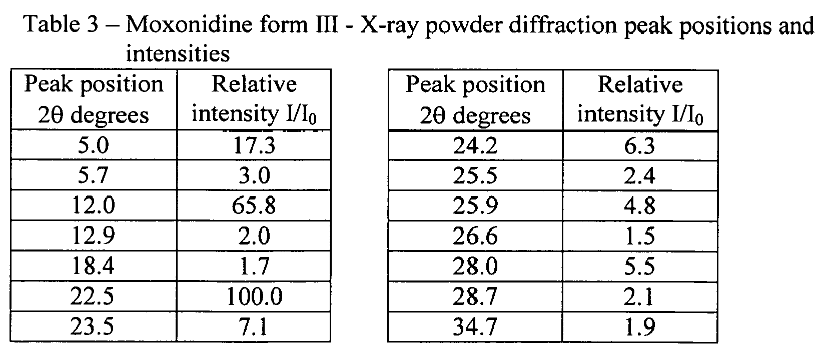

In another preferred embodiment of the present invention, the crystalline Moxonidine form III is an n-pentylamine solvate.

The process for preparing the crystalline Moxonidine form III, by crystallizing from n-pentylamine, comprises:
mixing Moxonidine, which is obtained by any method known in the art, e.g., by example 1, with n-pentylamine and heating to dissolution;
allowing the mixture to cool sufficiently; and
filtering off the thus obtained slurry and obtaining a solid;

In another embodiment of the present invention, a process is provided for preparing the crystalline Moxonidine form I by heating the crystalline Moxonidine form II or form III to elevated temperature.

According to another preferred embodiment of the present invention, there is provided a pharmaceutical composition comprising the crystalline solid comprising Moxonidine form I and pharmaceutically acceptable excipients and additives.

Although, the following examples illustrate the practice of the present invention in some of its embodiments, the examples should not be construed as limiting the scope of the invention. Other embodiments will be apparent to one skilled in the art from consideration of the specification and examples. It is intended that the specification, including the examples, is considered exemplary only, with the scope and spirit of the invention being indicated by the claims which follow.

### EXAMPLES

### EXPERIMENTAL METHODS

Moxonidine crystalline forms I, II and III of the present invention were characterized by powder X-ray diffraction, which produces a fingerprint of a particular crystalline form. Measurements of 2θ values typically are accurate to within ± 0.2 degrees 2θ. X-ray diffraction data were acquired using a PHILIPS X-ray diffractometer model PW1050-70. System description: K_{α1}=1.54178 A, voltage 40kV, current 28 mA, diversion slit=1°, receiving slit=0.2 mm, scattering slit=1° with a Graphite monochromator. Experiment parameters: pattern measured between 2θ = 4° and 2θ = 30° with 0.05° increments; count time was 0.5 second per increment.

The crystalline Moxonidine forms I, II and III of the present invention were also characterized by differential scanning calorimetry (DSC) run on TA instruments model Q1000, with Universal software version 3.88. Samples were analyzed inside crimped 40 µl Aluminum pans. Heating rate for all samples was 5°C/min.

The crystalline Moxonidine forms I, II and III of the present invention were also characterized by thermogravimetric analysis (TGA), a measure of the thermally induced weight loss of a material as a function of the applied temperature. Thermogravimetric analysis (TGA) was performed using a TA Instruments Q500 Thermal Analyzer with Universal Software (version 3.88). Samples were analyzed inside platinum baskets at heating rate of 5 °C/minute.

The particle size distribution of Moxonidine forms I, II and III of the present invention was measured by Malvern model Mastersizer 2000, equipped with Malvern Hydro G circulation cell. Carrier liquid: water. System description: Measuring range: from 0.02-2000µm. Accuracy: 1% on the Dv50. Light sources: Red light-helium neon laser. Blue light-solid state light source.

The ¹³C NMR spectra of Moxonidine form II of the present invention were taken using a Brucker DMX-15 digital FT NMR spectrometer equipped with a BL-4 cp/mass probe-head and a High Resolution/High Performance (HRHP) ¹H preamplifier for solids. The magic angle was optimized using KBr; 90° ¹H pulse, proton decoupling pulse and ¹³C shape pulse for Hartmann-Hahn matching were optimized using glycine. All spectra were calibrated with an external spectral reference where the value of 176.03 ppm was assigned to the carbonyl signal of the solid glycine standard.

### Example 1 - Preparation of crude Moxonidine by an improved process, as described in a copending application

A mixture of 4,6-dichloro-2-methyl-5-(1-acetyl-2-imidazolin-2-yl)-amino-pyrimidine (5 g, 0.0174 mol) and 85% potassium hydroxide powder (1.276 g, 0.019 mol, 1.1 equiv.) in methanol (40 ml) was stirred at ambient temperature for 30 hours. Then, water (50 ml) was added and the mixture was stirred for one hour. A colorless precipitate was collected by filtration, washed with water (3 × 10 ml) and 2-propanol (3 × 10 ml) and dried at 50° C overnight to give 3.5 g of crude Moxonidine in 83.5% yield, having a purity of 98.2% (by HPLC).

### Example 2 - Preparation of crystalline Moxonidine form I by crystallization of crude Moxonidine from DMSO

Crude Moxonidine (1.0 g) was mixed with DMSO (10 ml) and heated to a temperature of about 90°C to obtain a clear solution. The solution was cooled to ambient temperature and the thus formed crystals were obtained by filtration and dried under reduced pressure. Crystalline Moxonidine form I was obtained in 85% yield (0.85 g) having a purity of 99.9% (by HPLC). Particle size distribution was found to be: D(v,0.1)=5.3µ; D(v,0.5)=13.7µ; D(v,0.9)=31.2µ; D(v,0.95)=41.4µ.

### Examples 3-9 - Preparation of crystalline Moxonidine form I by crystallization of crude Moxonidine from various solvents

Crude Moxonidine (5.0 g), obtained as described in example 1, was mixed with a solvent, (20-400 ml as detailed in table 4) and heated to reflux temperature to obtain a solution. The solution was cooled to ambient temperature and the thus formed crystals were separated by filtration and dried under reduced pressure. The amount of the crystalline Moxonidine form I which was obtained in each experiment is detailed in table 4.

**Table 4 - Preparation of crystalline Moxonidine form I by crystallization of crude Moxonidine from different solvents**

| Ex No. | Solvent | Volume, ml | Volume per weight, ml/g | Moxonidine g | Yield % |
|---|---|---|---|---|---|
| 3 | DMF | 35 | 7 | 4.1 | 82 |
| 4 | DMA | 35 | 7 | 2.9 | 58 |
| 5 | NMP | 20 | 4 | 3.4 | 68 |
| 6 | Methanol | 115 | 23 | 2.4 | 48 |
| 7 | Ethanol | 200 | 40 | 4.25 | 85 |
| 8 | 1-Propanol | 120 | 24 | 3.75 | 75 |
| 9 | 2-Propanol | 400 | 80 | 4.25 | 85 |

### Example 10 - Preparation of crystalline Moxonidine form I by slurrying crude Moxonidine in acetone

Crude Moxonidine (5.0 g), obtained as described in example 1, was mixed with acetone (80 ml) and heated to reflux. The suspension was cooled to ambient temperature and the thus formed crystals were obtained by filtration and dried under reduced pressure. 4.4 g of crystalline Moxonidine form I were obtained in 88% yield. Particle size distribution was found to be: D(v,0.1)=98.4; D(v,0.5)=328.9µ; D(v,0.9)=605.8µ.

### Examples 11-28 - Preparation of crystalline Moxonidine form I by slurrying crude Moxonidine in other solvents

Crude Moxonidine (5.0 g), obtained as described in example 1, was mixed with a solvent, (300-400 ml as detailed in table 5) and heated to reflux temperature to obtain a suspension. The suspension was cooled to ambient temperature and the thus formed crystals were obtained by filtration and dried under reduced pressure. 4.2-4.95 g (as detailed in table 5) of crystallized Moxonidine form I was obtained in different yields.

**Table 5 - Preparation of crystalline Moxonidine form I by slurrying crude Moxonidine in different solvents**

| Ex. No. | Solvent | Volume of solvent, ml | Volume per weight, ml/g | Moxonidine g | Yield % |
|---|---|---|---|---|---|
| 11 | butyl acetate | 400 | 80 | 4.8 | 96 |
| 12 | cumene | 400 | 80 | 4.9 | 98 |
| 13 | cyclohexane | 400 | 80 | 4.9 | 98 |
| 14 | dichloromethane | 400 | 80 | 4.3 | 86 |
| 15 | diethyl ether | 400 | 80 | 4.9 | 98 |
| 16 | ethyl acetate | 350 | 70 | 4.5 | 90 |
| 17 | ethyl formate | 300 | 60 | 4.6 | 92 |
| 18 | heptane | 400 | 80 | 4.8 | 96 |
| 19 | isobutyl acetate | 300 | 60 | 4.6 | 92 |
| 20 | isopropyl acetate | 400 | 80 | 4.95 | 99 |
| 21 | methyl acetate | 400 | 80 | 4.6 | 92 |
| 22 | methylcyclohexane | 400 | 80 | 4.95 | 99 |
| 23 | methyl ethyl ketone (MEK) | 400 | 80 | 4.7 | 94 |
| 24 | methyl isobutyl ketone (MIBK) | 400 | 80 | 4.8 | 96 |
| 25 | pentane | 400 | 80 | 4.9 | 98 |
| 26 | propyl acetate | 300 | 60 | 4.8 | 96 |
| 27 | t-butyl methyl ether | 400 | 80 | 4.8 | 96 |
| 28 | tetrahydrofuran | 400 | 80 | 4.2 | 84 |

### Example 29 - Preparation of crystalline Moxonidine form II by crystallization of crude Moxonidine from ethylene glycol

Crude Moxonidine (1.0 g), obtained as described in example 1, was mixed with ethylene glycol (25 ml) and heated to a temperature of about 125°C. The solution was cooled to ambient temperature and the thus formed crystals of Moxonidine form II were obtained by filtration.

### Example 30 - Preparation of crystalline Moxonidine form III by crystallization of crude Moxonidine from n-pentylamine

Crude Moxonidine (1.5 g), obtained as described in example 1, was mixed with n-pentylamine (100 ml) and heated to reflux. The solution was cooled to ambient temperature and the thus formed crystals were collected by filtration to obtain 0.96 g of the crystalline Moxonidine form III.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A crystalline solid comprising Moxonidine form II, **characterized by** having X-ray powder diffraction pattern, wherein the strong diffraction peaks are at 7.5, 12.8, 13.0, 18.0, 22.7, 24.0, and 25.8 ±0.2 degrees 2θ.

2. The crystalline solid comprising Moxonidine form II of claim 1, further **characterized by** being a solvate, wherein the two ¹³C NMR signals at 62.5 and 63.4 ppm in the spectrum are attributed to ethylene glycol, while one of these signals arises from a molecule inside the crystal lattice and the other one is a surface material.

3. A process for preparing the crystalline Moxonidine form II, comprising:
mixing Moxonidine, which is obtained as per any method known in the art, e.g., by example 1, with ethylene glycol and heating to dissolution;
allowing the mixture to cool sufficiently; and
filtering off the thus obtained slurry and obtaining a solid;

4. A crystalline solid comprising Moxonidine form III **characterized by** a unique X-ray powder diffraction pattern, wherein the strong diffraction peaks at 5.0, 12.0, and 22.5±0.2 degrees 2θ are most characteristic of this form.

5. The crystalline solid comprising Moxonidine form III of claim 4, further **characterized by** being an n-pentylamine solvate.

6. A process for preparing the crystalline Moxonidine form III, by crystallizing from n-pentylamine, comprising:
mixing Moxonidine, which is obtained by any method known in the art, e.g., by example 1, with n-pentylamine and heating to dissolution; allowing the mixture to cool sufficiently; and
filtering off the thus obtained slurry and obtaining a solid.

7. A process for preparing the crystalline Moxonidine form I by heating the crystalline solid comprising Moxonidine form II or form III at elevated temperature.

8. Moxonidine form I in which 90% of the particles have a diameter of 60 microns or less.

9. Moxonidine form I in which 90% of the particles have a diameter of about 32 microns or less.

10. Moxonidine form I in which 50% of the particles have a diameter of 50 microns or less,

11. Moxonidine form I in which 50% of the particles have a diameter of about 25 microns or less.

12. A pharmaceutical composition comprising the crystalline solid Moxonidine form I and pharmaceutically acceptable excipients and additives.

## Amended claims

### Amended claims in accordance with Rule 137(2) EPC.

**1.** Moxonidine form I **characterized by** having a X-ray powder diffraction pattern wherein the strong diffraction peaks are at 12.1, 13.4, 18.4, 22.6, 23.7, 25.5, 27.1 and 28.2±0.2 degrees 2θ and an exothermic peak at about 227°C in its DSC curve.

**2.** Moxonidine form I according to claim 1 in which 90% of the particles have a diameter of 60 microns or less.

**3.** Moxonidine form I according to claim 1 in which 90% of the particles have a diameter of about 32 microns or less.

**4.** Moxonidine form I according to claim 1 in which 50% of the particles have a diameter of 50 microns or less.

**5.** Moxonidine form I according to claim 1 in which 50% of the particles have a diameter of about 25 microns or less.

**6.** A pharmaceutical composition comprising the crystalline solid Moxonidine form I according to claims 1 to 5 and pharmaceutically acceptable excipients and additives.

**7.** A process for preparing the crystalline Moxonidine form I according to claim 1, which comprises:
(i) slurrying or dissolving Moxonidine obtained by any method known in the art in a solvent optionally at an elevated temperature;
(ii) allowing the mixture to cool; and
(iii) collecting the obtained crystals by filtration and drying.

**8.** The process according to claim 7, wherein the solvent is selected from the group consisting of water; glycols; polar solvents selected from the group consisting of dimethyl sulfoxide (DMSO), N,N-dimethylformamide (DMF), N,N-dimethylacetamide (DMA), and N-methyl-2-pyrrolidone (NMP); alcohols; R₁COOR₂ esters, wherein R₁ is C₁-C₅ alkyl and R₂ is C₁-C₅ alkyl; C₁-C₆ ketones; halogenated solvents; ethers; aromatic solvents and hydrocarbons.

**9.** The process according to claim 8, wherein the alcohols are selected from the group consisting of methanol, ethanol, 1-propanol, 2-propanol, 1-butanol, 2-butanol, 2-methyl-1-propanol, 3-methyl-1-propanol, and n-amyl-alcohol; the R₁COOR₂ esters are selected from the group consisting of ethyl formate, methyl acetate, ethyl acetate, n-propyl acetate, isopropyl acetate, n-butyl acetate, isobutyl acetate, and tert-butyl acetate; the C₁-C₆ ketones are selected from the group consisting of acetone, methyl ethyl ketone (MEK), diethyl ketone, methyl propyl ketone, and methyl isobutyl ketone (MIBK); the halogenated solvent is dichloromethane; wherein the ethers are selected from the group consisting of diethyl ether, tert-butyl methyl ether, and tetrahydrofuran (THF); wherein the aromatic solvents are selected from anisole, cumene and toluene; and wherein the hydrocarbons are selected from the group consisting of pentane, hexane, heptane, cyclohexane and methylcyclohexane, and mixtures thereof.

**10.** A process for preparing the crystalline Moxonidine form I according to claim 1, which comprises heating a crystalline solid comprising Moxonidine form II or form III at elevated temperature, wherein said Moxonidine form II is **characterized by** having X-ray powder diffraction pattern in which the strong diffraction peaks are at 7.5, 12.8, 13.0, 18.0, 22.7, 24.0, and 25.8 ± 0.2 degrees 2θ and said Moxonidine form III is **characterized by** a unique X-ray powder diffraction pattern in which the strong diffraction peaks at 5.0, 12.0, and 22.5 ± 0.2 degrees 2θ are most characteristic.
